# EUROPEAN PATENT SPECIFICATION

(11) **EP 2 903 597 B1**
(45) Date of publication and mention of the grant of the patent: **18.12.2019**
(21) Application number: 13844278.5
(22) Date of filing: 03.10.2013
(51) Int. Cl.: C12Q 1/6886

(54) **USE OF MICROVESICLES IN DIAGNOSIS, PROGNOSIS, AND TREATMENT OF MEDICAL DISEASES AND CONDITIONS**
VERWENDUNG VON MIKROVESIKELN BEI DER DIAGNOSE, PROGNOSE UND BEHANDLUNG VON KRANKHEITEN UND MEDIZINISCHEN LEIDEN
UTILISATION DE MICROVÉSICULES DANS LE DIAGNOSTIC, LE PRONOSTIC ET LE TRAITEMENT DE MALADIES ET D'ÉTATS MÉDICAUX

(30) Priority: 03.10.2012 US 201261709337 P
(43) Date of publication of application: 12.08.2015
(73) Proprietor: Exosome Diagnostics Inc., New York, NY 10032 (US)
(72) Inventor: COMPER, Wayne, Cambridge, MA 02139 (US); RAMACHANDRAN, Aparna, Iselin, NJ 08830 (US); YAN, Haoheng, Hastings on Hudson, NY 10706 (US); RUSSO, Leileata, M., Belmont, MA 02478 (US); SKOG, Johan Karl Olov, Charlestown, MA 02129 (US)
(74) Representative: Cooley (UK) LLP
(86) International application number: PCT/US2013/063292
(87) International publication number: WO 2014/055775

(56) References cited:
- WO-A1-2012/170711
- WO-A1-2013/028788
- US-A1- 2010 004 253
- US-A1- 2010 196 426
- US-A1- 2010 196 426
- APARNA RAM ACHANDRAN ET AL: "Detection of BRAF mutations in serum/plasma microvesicles (exosomes) of malignant melanoma patients.", MOL CANCER THER, vol. 10, no. 11 Suppl, 1 January 2011 (2011-01-01), page C139, XP055275845,
- Stefan Bentink: "Entwicklung eines diagnostischen Assays zur Identifizierung von Mutationen in verschiedenen Genen als Biomarker beim malignen Melanom (PM20)", , 6 March 2012 (2012-03-06), XP055275878, Retrieved from the Internet: URL:http://www.bio-m.org/fileadmin/user_up load/Veranstaltungen/2012/m4_Symposium/1-5 -Bentink-PM20_ok.pdf [retrieved on 2016-05-27]
- DIEDERICK DUIJVESZ ET AL: "Exosomes as Biomarker Treasure Chests for Prostate Cancer", EUROPEAN UROLOGY, ELSEVIER BV, NL, vol. 59, no. 5, 20 December 2010 (2010-12-20), pages 823-831, XP028160348, ISSN: 0302-2838, DOI: 10.1016/J.EURURO.2010.12.031 [retrieved on 2010-12-22]
- MARCUS C RAVNAN ET AL: "Vemurafenib in Patients WithMutation Positive Advanced Melanoma", CLINICAL THERAPEUTICS, EXCERPTA MEDICA, PRINCETON, NJ, US, vol. 34, no. 7, 6 June 2012 (2012-06-06), pages 1474-1486, XP028405379, ISSN: 0149-2918, DOI: 10.1016/J.CLINTHERA.2012.06.009 [retrieved on 2012-06-20]
- SCHWARZENBACH ET AL.: 'Cell-free nucleic acids as biomarkers in cancer patients' NATURE REVIEW CANCER vol. 11, 12 May 2011, pages 426 - 437, XP055247315
- SHI ET AL.: 'Combinatorial Treatments That Overcome PDGFRbeta-Driven Resistance of Melanoma Cells to V600EB-RAF Inhibition' CANCER RES vol. 71, no. 15, 01 August 2011, pages 5067 - 5074, XP002695801
- JOSEPH ET AL.: 'The RAF inhibitor PLX4032 inhibits ERK signaling and tumor cell proliferation in a V600E BRAFselective manner' PNAS vol. 107, no. 33, 17 August 2010, pages 14903 - 14908, XP055098494
- WAGLE ET AL.: 'Dissecting Therapeutic Resistance to RAF Inhibition in Melanoma by Tumor Genomic Profiling' J CLIN ONCOL vol. 29, no. 22, 07 March 2011, pages 3085 - 3096, XP009151363

## Description

### BACKGROUND OF THE INVENTION

Serine/threonine-protein kinase BRAF, a downstream effector of the RAS oncogene along the MEK/ERK signaling pathway, has emerged as an important biological marker for diagnosis, prognosis and therapeutic guidance for human cancers. The high prevalence of mutant BRAF V600E implies that the mutation is an important 'driver' or 'codriver' in the development of a subset of these cancers. Moreover, cancers with a BRAF mutation are generally more aggressive than their counterparts without the mutation. Accordingly, mutant BRAF has been a highly attractive target for precision cancer therapy. Therefore, detection of BRAF for use in diagnosis, prognosis and therapeutic guidance has become increasingly important in clinical applications.

Current techniques to detect cancer mutation profiles, such as BRAF mutations, include the analysis of biopsy samples and the non-invasive analysis of mutant tumor DNA fragments circulating in bodily fluids such as blood (Diehl et al., 2008). The former method is invasive, complicated and potentially harmful to subjects. Moreover, in the intrusive biopsy procedure, tissue samples are taken from a limited area and therefore, may give false positives or false negatives, especially in tumors which are heterogeneous and/or dispersed within normal tissue. The latter method inherently lacks sensitivity due to the extremely low copy number of mutant cancer DNA in bodily fluid (Gormally et al., 2007). Therefore, a non-intrusive and sensitive diagnostic method for detecting BRAF mutations would be highly desirable.

### SUMMARY OF THE INVENTION

The present disclosure addresses the need for non-intrusive and highly accurate diagnostic methods for detecting BRAF mutations. In general, the present disclosure features methods for detecting BRAF mutations from DNA and/or RNA isolated from microvesicles from a biological sample.

The present invention features a method for diagnosing cancer in a subject comprising isolating a microvesicle fraction from a biological sample from the subject, extracting DNA and/or RNA from the microvesicles, and detecting the presence or absence of a BRAF mutation in the extracted DNA and/or RNA, where the presence of the BRAF mutation in the extracted DNA and/or RNA indicates the presence of cancer in the subject or a higher predisposition of the subject to develop cancer.

The present invention features a method for determining a therapeutic regimen for treatment of a subject suffering from cancer comprising isolating a microvesicle fraction from a biological sample from the subject, extracting DNA and/or RNA from the microvesicles, and detecting the presence or absence of a BRAF mutation in the extracted DNA and/or RNA, wherein the presence of the BRAF mutation in the extracted DNA and/or RNA indicates the use of a therapeutic regimen that comprises at least one kinase inhibitor. In some embodiments, the kinase inhibitor is a RAF inhibitor or a MEK inhibitor. In some embodiments, the RAF inhibitor is a BRAF-specific inhibitor. In some preferred embodiments, the therapeutic regimen comprises a drug that targets mutated BRAF or downstream signaling from mutated BRAF. For example, the therapeutic regimen comprises vemurafenib or dabrafenib. In some embodiments, the cancer is melanoma, thyroid cancer, colorectal cancer, ovarian cancer, breast cancer, brain cancer, pancreas cancer, lung cancer, lymphoma or leukemia.

The present invention features a method wherein the BRAF mutation is an activating mutation.

The present invention features a method wherein the BRAF mutation encodes a mutant BRAF polypeptide wherein the mutant BRAF polypeptide is V600E.

The present invention features a method wherein the BRAF mutation is T1799A.

The present invention features a method wherein the biological sample is a bodily fluid sample. In some aspects, the bodily fluid sample is plasma, serum, cerebrospinal fluid, or ascites fluid. In other preferred embodiments, the bodily fluid sample is bronchoalveolar lavag (BAL) and cyst fluid. In some aspects, the bodily fluid sample is within the range of 2 to 20 ml.

### BRIEF DESCRIPTION OF THE FIGURES

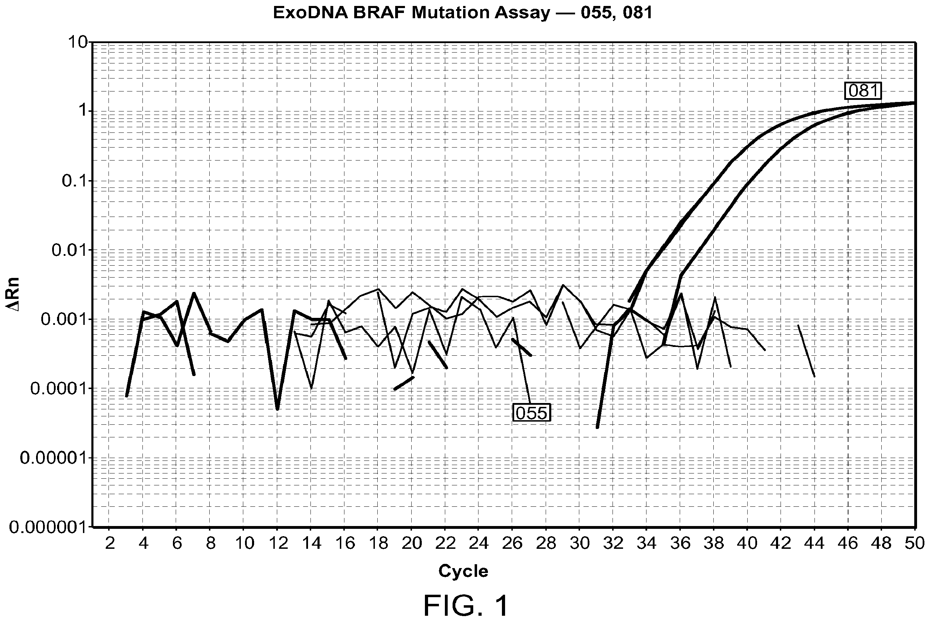

**Figure 1** is a graphical representation of the real-time PCR data for sample 081 (dark grey) and 055 (light grey). The amplification curves represent triplicates of the QPCR assay on DNA extracted from sample 081. A BRAF mutation (V600E) was detected in sample 081, but not in sample 055 in accordance with biopsy data from the tumor tissue. ΔRn represents the reporter signal normalized to a reference fluorescence signal and the baseline fluorescence; log (ΔRn) is plotted against PCR cycle number.

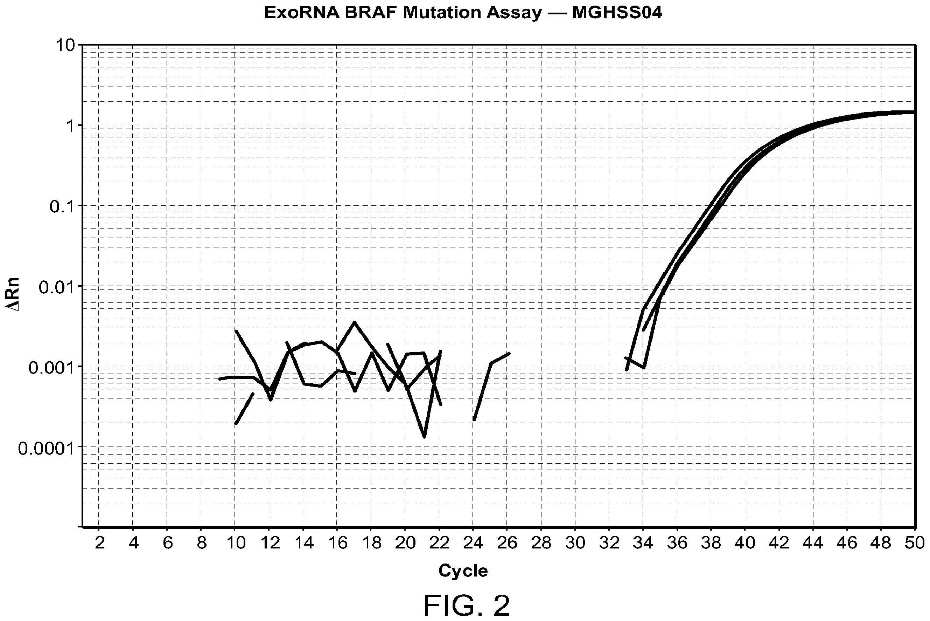

**Figure 2** is a graphical representation of the real-time PCR data for RNA extracted from sample MGHSS04. A BRAF mutation was detected in accordance with biopsy data from the tumor tissue. ΔRn represents the reporter signal normalized to a reference fluorescence signal and the baseline fluorescence; log (ΔRn) is plotted against PCR cycle number.

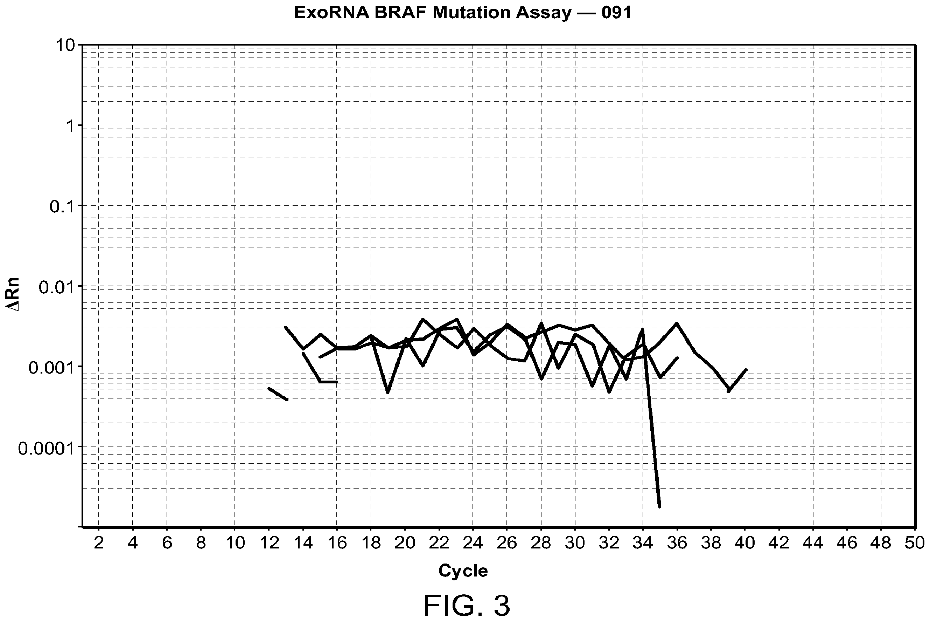

**Figure 3** is a graphical representation of the real-time PCR data for RNA extracted from sample 091. No mutation was detected in sample 091 in accordance with biopsy data from the tumor tissue. ΔRn represents the reporter signal normalized to a reference fluorescence signal and the baseline fluorescence; log (ΔRn) is plotted against PCR cycle number.

### DETAILED DESCRIPTION OF THE INVENTION

Microvesicles are shed by eukaryotic cells, or budded off of the plasma membrane, to the exterior of the cell. These membrane vesicles are heterogeneous in size with diameters ranging from about 10nm to about 5000 nm. The small microvesicles (approximately 10 to 1000nm, and more often 30 to 200 nm in diameter) that are released by exocytosis of intracellular multivesicular bodies are referred to in the art as "exosomes". The methods and compositions described herein are equally applicable to microvesicles of all sizes; preferably 30 to 800 nm.

In some of the literature, the term "exosome" also refers to protein complexes containing exoribonucleases which are involved in mRNA degradation and the processing of small nucleolar RNAs (snoRNAs), small nuclear RNAs (snRNAs) and ribosomal RNAs (rRNA) (Liu et al., 2006b; van Dijk et al., 2007). Such protein complexes do not have membranes and are not "microvesicles" or "exosomes" as those terms are used here in.

Recently, studies have revealed that nucleic acids within microvesicles have a role as biomarkers. The use of nucleic acids extracted from mirovesicles is considered to potentially circumvent the need for biopsies, highlighting the enormous diagnostic potential of microvesicle biology (Skog et al., 2008).

Stefan Bentink: "Entwicklung eines diagnostischen Assays zur Identifizierung von Mutationen in verschiedenan Genen als Biomarker beim malignen Melanon (PM20)" describes the concept of an essay in which the microvesile fraction from the patient sample (blood, urine or biopsy) is isolated and the RNA is analysed for the presence of the BRAF V6OOE mutation, which is known to be caused by the T1799A variation. Ram achandran et. al, Molecular Cancer Therapeutics, November 2011, Vo. 10, page C139 and US 2010/196426 A1 both disclose methods to isolate nucleic acids from exosomes. The methods described herein feature the use of nucleic acids extracted from microvesicles for detection of BRAF mutations for use in diagnosing, prognosing the presence of a disease or medical conditions in a subject, or for assessing or determining the treatment regimen for a subject suffering from a disease or medical condition.

### BRAF

RAF kinases are highly conserved serine/threonine kinases that are key components of the mitogen-activated protein (MAP) kinase pathway, a signal-transduction pathway that plays a fundamental role in the regulation of gene expression, cell growth, proliferation, differentiation, and programmed cell death. The MAP kinase pathway is conserved in eukaryotes and functions to transduce extracellular signals, such as hormones, cytokines, and various growth factors, via receptors and phosphorylation cascades to the nucleus for activation of transcription factors. The signaling pathway is initiated through activation of receptor tyrosine kinases by extracellular mitogenic signals. The receptor tyrosine kinase activates Ras, a GTPase, which causes membrane recruitment and activation of RAF proteins. In turn, activation of RAF leads to the phosphorylation and subsequent activation of the protein kinase MEK. MEK then phosphorylates ERK, which can directly and indirectly activate transcription factors, leading to the expression of various regulatory genes involved in cell proliferation and survival.

BRAF, also known as v-raf murine sarcoma viral oncogene homolog B1, B-RAF, BRAF1, B-RAF1, NS7, and RAFB1, is a member of the RAF family of serine/threonine protein kinases. This family consists of 3 highly conserved kinases: ARAF (or A-RAF), CRAF (RAF-1 or C-RAF), and BRAF. As used herein, "BRAF" encompasses all known human BRAF homologues and variants, as well as other nucleic acids and polypeptides which exhibit 70%, 75%, 80%, 85%, 90%, 95%, 98%, or 99% homology to BRAF. BRAF may be identified as comprising the nucleic acid sequence shown at Genbank Accession No. NM_004333 (SEQ ID NO:1), wherein the start and stop codons are italicized and underlined and the common oncogenic mutation that results in a mutation at amino acid 600 is underlined and in bold:

CGCCTCCCTTCCCCCTCCCCGCCCGACAGCGGCCGCTCGGGCCCCGGCTCTCGGTTATAAGATGGCGGCGCTG
AGCGGTGGCGGTGGTGGCGGCGCGGAGCCGGGCCAGGCTCTGTTCAACGGGGACATGGAGCCCGAGGCCGGCG
CCGGCGCCGGCGCCGCGGCCTCTTCGGCTGCGGACCCTGCCATTCCGGAGGAGGTGTGGAATATCAAACAAAT
GATTAAGTTGACACAGGAACATATAGAGGCCCTATTGGACAAATTTGGTGGGGAGCATAATCCACCATCAATA
TATCTGGAGGCCTATGAAGAATACACCAGCAAGCTAGATGCACTCCAACAAAGAGAACAACAGTTATTGGAAT
CTCTGGGGAACGGAACTGATTTTTCTGTTTCTAGCTCTGCATCAATGGATACCGTTACATCTTCTTCCTCTTC
TAGCCTTTCAGTGCTACCTTCATCTCTTTCAGTTTTTCAAAATCCCACAGATGTGGCACGGAGCAACCCCAAG
TCACCACAAAAACCTATCGTTAGAGTCTTCCTGCCCAACAAACAGAGGACAGTGGTACCTGCAAGGTGTGGAG
TTACAGTCCGAGACAGTCTAAAGAAAGCACTGATGATGAGAGGTCTAATCCCAGAGTGCTGTGCTGTTTACAG
AATTCAGGATGGAGAGAAGAAACCAATTGGTTGGGACACTGATATTTCCTGGCTTACTGGAGAAGAATTGCAT
GTGGAAGTGTTGGAGAATGTTCCACTTACAACACACAACTTTGTACGAAAAACGTTTTTCACCTTAGCATTTT
GTGACTTTTGTCGAAAGCTGCTTTTCCAGGGTTTCCGCTGTCAAACATGTGGTTATAAATTTCACCAGCGTTG
TAGTACAGAAGTTCCACTGATGTGTGTTAATTATGACCAACTTGATTTGCTGTTTGTCTCCAAGTTCTTTGAA
CACCACCCAATACCACAGGAAGAGGCGTCCTTAGCAGAGACTGCCCTAACATCTGGATCATCCCCTTCCGCAC
CCGCCTCGGACTCTATTGGGCCCCAAATTCTCACCAGTCCGTCTCCTTCAAAATCCATTCCAATTCCACAGCC
CTTCCGACCAGCAGATGAAGATCATCGAAATCAATTTGGGCAACGAGACCGATCCTCATCAGCTCCCAATGTG
CATATAAACACAATAGAACCTGTCAATATTGATGACTTGATTAGAGACCAAGGATTTCGTGGTGATGGAGGAT
CAACCACAGGTTTGTCTGCTACCCCCCCTGCCTCATTACCTGGCTCACTAACTAACGTGAAAGCCTTACAGAA
ATCTCCAGGACCTCAGCGAGAAAGGAAGTCATCTTCATCCTCAGAAGACAGGAATCGAATGAAAACACTTGGT
AGACGGGACTCGAGTGATGATTGGGAGATTCCTGATGGGCAGATTACAGTGGGACAAAGAATTGGATCTGGAT
CATTTGGAACAGTCTACAAGGGAAAGTGGCATGGTGATGTGGCAGTGAAAATGTTGAATGTGACAGCACCTAC
ACCTCAGCAGTTACAAGCCTTCAAAAATGAAGTAGGAGTACTCAGGAAAACACGACATGTGAATATCCTACTC
TTCATGGGCTATTCCACAAAGCCACAACTGGCTATTGTTACCCAGTGGTGTGAGGGCTCCAGCTTGTATCACC
ATCTCCATATCATTGAGACCAAATTTGAGATGATCAAACTTATAGATATTGCACGACAGACTGCACAGGGCAT
GGATTACTTACACGCCAAGTCAATCATCCACAGAGACCTCAAGAGTAATAATATATTTCTTCATGAAGACCTC
ACAGTAAAAATAGGTGATTTTGGTCTAGCTACAGTGAAATCTCGATGGAGTGGGTCCCATCAGTTTGAACAGT
TGTCTGGATCCATTTTGTGGATGGCACCAGAAGTCATCAGAATGCAAGATAAAAATCCATACAGCTTTCAGTC
AGATGTATATGCATTTGGAATTGTTCTGTATGAATTGATGACTGGACAGTTACCTTATTCAAACATCAACAAC
AGGGACCAGATAATTTTTATGGTGGGACGAGGATACCTGTCTCCAGATCTCAGTAAGGTACGGAGTAACTGTC
CAAAAGCCATGAAGAGATTAATGGCAGAGTGCCTCAAAAAGAAAAGAGATGAGAGACCACTCTTTCCCCAAAT
TCTCGCCTCTATTGAGCTGCTGGCCCGCTCATTGCCAAAAATTCACCGCAGTGCATCAGAACCCTCCTTGAAT
CGGGCTGGTTTCCAAACAGAGGATTTTAGTCTATATGCTTGTGCTTCTCCAAAAACACCCATCCAGGCAGGGG
GATATGGTGCGTTTCCTGTCCACTGAAACAAATGAGTGAGAGAGTTCAGGAGAGTAGCAACAAAAGGAAAATA
AATGAACATATGTTTGCTTATATGTTAAATTGAATAAAATACTCTCTTTTTTTTTAAGGTGAACCAAAGAACA
CTTGTGTGGTTAAAGACTAGATATAATTTTTCCCCAAACTAAAATTTATACTTAACATTGGATTTTTAACATC
CAAGGGTTAAAATACATAGACATTGCTAAAAATTGGCAGAGCCTCTTCTAGAGGCTTTACTTTCTGTTCCGGG
TTTGTATCATTCACTTGGTTATTTTAAGTAGTAAACTTCAGTTTCTCATGCAACTTTTGTTGCCAGCTATCAC
ATGTCCACTAGGGACTCCAGAAGAAGACCCTACCTATGCCTGTGTTTGCAGGTGAGAAGTTGGCAGTCGGTTA
GCCTGGGTTAGATAAGGCAAACTGAACAGATCTAATTTAGGAAGTCAGTAGAATTTAATAATTCTATTATTAT
TCTTAATAATTTTTCTATAACTATTTCTTTTTATAACAATTTGGAAAATGTGGATGTCTTTTATTTCCTTGAA
GCAATAAACTAAGTTTCTTTTTATAAAAA

BRAF may be identified as a polypeptide having the sequence of Genbank Accession No. NP_004324 (SEQ ID NO:2), wherein the oncogenic mutation at amino acid 600 is underlined and in bold:

The BRAF kinase comprises a Ras-binding domain and a protein kinase domain. The BRAF kinase domain exhibits characteristic bilobal architecture, with the small N-terminal lobe (N-lobe) and large C-terminal lobe (C-lobe) separated by a catalytic cleft. The N-lobe contains a glycine-rich ATP-phosphate-binding loop (P-loop), which anchors and orients ATP, which is critical for the kinase activity. In the inactive conformation, the catalytic cleft is rendered inaccessible, however upon activation by Ras, the kinase undergoes a conformational change such that the catalytic cleft if accessible and BRAF is active. Active BRAF signals through MEK to activate ERK, which, in turn, activates downstream transcription factors to induce a range of biochemical processes including cell differentiation, proliferation, growth, and apoptosis.

### BRAF mutation and cancer

The MAPK pathway is mutated in an estimated 30% of all cancers, with mutations in the BRAF gene found in approximately 7% of all cancers (Garnett et al., 2004, Davies et al., 2002). Importantly, BRAF has been found to be mutated in a wide range of cancers, including 40-70% of malignant melanomas, which is the 6^{th} most common cancer, 45% of papillary thyroid cancer, 10% of colorectal cancer, and has also been identified in ovarian, breast, and lung cancer, and lymphoma and leukemias.

Activating mutations in BRAF were first described by the Sanger Institute in 2002 (Davies et al., 2002). Currently, there are approximately 40 different mutations that have been identified in the BRAF gene associated with human cancer. The most common of the BRAF mutations is the single base change from a thymine to an adenine at position 1799 (formerly referred to as the 1796 position), which is highlighted in SEQ ID NO:1, resulting in a substitution of glutamic acid (E) for valine (V) at position 600 of the amino acid sequence (formerly referred to as the 599 position), which is highlighted in SEQ ID NO:2. According to the Catalogue of Somatic Mutations in Cancer (COS-MIC) database, this mutation currently accounts for up to 97% of all BRAF mutations. Cancer-associated mutations have been mostly identified in the kinase domain.

The present description discloses a method for determining the presence or absence of one or more mutations in the BRAF nucleic acid. Preferably, the mutation is an activating or oncogenic mutation. Preferably, the mutation is a substitution. The mutation can also be one or more substitutions, insertions, deletions, or rearrangements or a combination thereof of the BRAF nucleic acid. The mutation may be a substitution mutation at position 1799 of the BRAF nucleic acid. Preferably, the mutation is a single base change or missense mutation, at position 1799 of the BRAF nucleic acid sequence, wherein the T is mutated to an A.

The mutation of the BRAF nucleic acid may encode a mutant BRAF polypeptide. Preferably, the mutant BRAF polypeptide is V600E (also known as V599E). Other preferred mutant BRAF polypeptides include: V600K, V600D and V600R. The mutant BRAF polypeptides may additionally include, mutations at the following amino acid positions: 439, 440, 443, 444, 453, 456, 459, 460, 462, 464, 466, 467, 468, 469, 471, 472, 475, 485, 581, 582, 583, 584, 585, 586, 587, 588, 589, 590, 600, 601, 602, 603, 604, 605, 606, 607, 608, 609, 610, 611, 612, 614, 615, 616, 617, 618, 619 or a combination thereof. BRAF mutations include any nucleic acids that encode a mutant BRAF polypeptide as disclosed herein.

The present disclosure provides methods for detecting the presence or absence of BRAF mutant nucleic acids from nucleic acids extracted from microvesicles from a biological sample for the use of diagnosing or prognosing the presence of a disease or medical condition in a subject. The present disclosure also provides methods for detecting the presence or absence of BRAF mutant nucleic acids from nucleic acids extracted from microvesicles for the use of assessing or determining the treatment regimen for a subject suffering from a disease or medical condition.

Germline mutations in BRAF are also associated with developmental diseases, such as LEOPARD, Noonan, and cardiofaciocutaenous syndromes. All three syndromes are associated with activating mutations of BRAF, though often less activating than the cancer-associated V600E mutation.

Due to the high prevalence of mutations in the RAS/BRAF/MEK/ERK pathway found in cancers, therapeutics targeting this particular pathway and its signaling components have been the subject of intense research. Kinase inhibitors have been shown to have some success in treating some cancers. RAF inhibitors, which include BRAF-specific inhibitors, have shown some efficacy in treating cancer patients. Examples of BRAF-specific inhibitors include: GDC-0879 and PLX4720. Other ways of targeting cancers that exhibit BRAF activating mutations include targeting the downstream effectors of BRAF, such as MEK, e.g., by using a MEK inhibitor.

BRAF, as used herein, refers to the gene (*i.e.,* nucleotide sequence that encodes the BRAF protein) or the protein.

### Microvesicles as Diagnostic And/Or Prognostic Tools

Certain aspects of the present disclosure are based on the finding that microvesicles are secreted by tumor cells and circulating in bodily fluids. The number of microvesicles increases as the tumor grows. The concentration of the microvesicles in bodily fluids is proportional to the corresponding active tumor load. The bigger the tumor load, the higher the concentration of microvesicles in bodily fluids. The nucleic acids found within these microvesicles, as well as other contents of the microvesicles such as angiogenic proteins, can be used as valuable biomarkers for tumor diagnosis, characterization and prognosis by providing a genetic profile. Importantly, biomarkers with mutations, such as the mutation that encodes a BRAF polypeptide with a V600E mutation, can be accurately detected from nucleic acids isolated from microvesicles using the methods described herein. Contents within these microvesicles can also be used to monitor tumor progression over time by analyzing if other mutations are acquired during tumor progression as well as if the levels of certain mutations are becoming increased or decreased over time or over a course of treatment.

The present disclosure relates to methods for detecting, diagnosing, monitoring, treating or evaluating a disease or other medical condition in a subject comprising the steps of, isolating microvesicles from a bodily fluid of a subject, and analyzing one or more nucleic acids contained within the exosomes. The nucleic acids are analyzed qualitatively and/or quantitatively, and the results are compared to results expected or obtained for one or more other subjects who have or do not have the disease or other medical condition. The presence of a difference in microvesicular nucleic acid content of the subject, as compared to that of one or more other individuals, can indicate the presence or absence of, the progression of (e.g., changes of tumor size and tumor malignancy), or the susceptibility to or predisposition for a disease or other medical condition in the subject. The presence of certain nucleic acids in the microvesicles isolated from the subject can be used to help determine the treatment regimen to be used that would be most efficacious.

The disclosure features a method for diagnosing a disease or other medical condition in a subject. The disease or medical condition may be cancer. The method comprises isolating a microvesicle fraction from a biological sample from the subject, extracting DNA and/or RNA from the microvesicles, and detecting the presence or absence of a BRAF mutation in the extracted DNA and/or RNA. The BRAF mutation may be any mutation disclosed herein; preferably the BRAF mutation is V600E. The presence of the BRAF mutation indicates the presence of the disease or medical condition or a higher predisposition of the subject to develop the disease or medical condition.

The disclosure features a method for determining a therapeutic regiment for treatment of a subject suffering from a disease or other medical condition. The disease or medical condition may be cancer. The method comprises isolating a miscrovesicle fraction from a biological sample from the subject, extracting DNA and/or RNA from the microvesicles, and detecting the presence or absence of a BRAF mutation in the extracted DNA and/or RNA. The BRAF mutation may be any mutation disclosed herein; preferably the BRAF mutation is V600E. The presence of the BRAF mutation in the extracted DNA and/or RNA indicates that the tumor initiation or progression is dependent on the oncogenic or activating mutation of BRAF. Accordingly, the presence of the BRAF mutation indicates that the subject may benefit from a therapeutic regimen that comprises a kinase inhibitor, particularly, a RAF or a BRAF inhibitor. Absence of the BRAF mutation indicates that the subject may not benefit from a therapeutic regimen that comprises a kinase inhibitor, such as a RAF or BRAF inhibitor.

Drugs that treat cancers driven by mutated BRAF have been and are currently being developed. Two of these drugs, vemurafenib and dabrafenib were approved by the U.S. Food and Drug Administration for treatment of late-stage melanoma. Vemurafenib is a B-raf inhibitor that interrupts the B-Raf/MEK/ERK pathway driven by mutated BRAF (*i.e.,* V600E). Similarly, Dabrafenib is a potent inhibitor of mutated BRAF (*i.e.,* V600E/K). Therefore, subjects that have been identified as having mutated BRAF using the methods described herein may benefit from a therapeutic regimen comprising an agent that targets or inhibits mutated BRAF and/or its downstream signaling. For example, the subject may benefit from a therapeutic regimen comprising vemurafenib or dabrafenib, while subjects that do not have mutated BRAF may not benefit from a therapeutic regimen comprising vemurafenib or dabrafenib.

The methods disclosed herein can be used to assess the responsiveness of a subject to a therapeutic regimen. For example, a BRAF mutation can be detected by the methods disclosed herein, and the presence of the BRAF mutation indicates that the subject may be responsive to a particular therapeutic regimen. For example, the therapeutic regimen comprises a kinase inhibitor, such as a RAF or a BRAF inhibitor, or a drug that targets mutated BRAF and/or the signaling downstream from mutated BRAF. A determination of responsiveness of a subject to a particular therapeutic regimen is useful for the selection of a therapeutic regimen.

Indeed, the isolation methods and techniques described herein provide the following heretofore unrealized advantages: 1) the opportunity to selectively analyze disease-or tumor- specific nucleic acids, which may be realized by isolating disease- or tumor- specific microvesicles apart from other microvesicles within the fluid sample; 2) significantly higher yield of nucleic acid species with higher sequence integrity as compared to the yield/integrity obtained by extracting nucleic acids directly from the fluid sample; 3) scalability, e.g. to detect nucleic acids expressed at low levels, the sensitivity can be increased by pelleting more microvesicles from a larger volume of serum; 4) purer nucleic acids in that protein and lipids, debris from dead cells, and other potential contaminants and PCR inhibitors are excluded from the microvesicle pellets before the nucleic acid extraction step; and 5) more choices in nucleic acid extraction methods as microvesicle pellets are of much smaller volume than that of the starting serum, making it possible to extract nucleic acids from these microvesicle pellets using small volume column filters.

The microvesicles are preferably isolated from a sample taken of a bodily fluid from a subject. As used herein, a "bodily fluid" refers to a sample of fluid isolated from anywhere in the body of the subject, preferably a peripheral location, including but not limited to, for example, blood, plasma, serum, urine, sputum, spinal fluid, pleural fluid, nipple aspirates, lymph fluid, fluid of the respiratory, intestinal, and genitourinary tracts, tear fluid, saliva, breast milk, fluid from the lymphatic system, semen, cerebrospinal fluid, intra-organ system fluid, ascitic fluid, bronchoalveolar lavage (BAL), cyst fluid, tumor cyst fluid, amniotic fluid and combinations thereof. Preferably, the bodily fluid is plasma, serum, cerebrospinal fluid, ascites fluid, bronchoaveolar lavage, or cyst fluid. The bodily fluid sample may be within the range of 2-20 ml. It may be preferable to use a larger volume of sample for increased accuracy in detecting rare genetic mutations, such as the BRAF mutation described herein. The bodily fluid sample may be within the range of 1 to 25 ml, for example, from 2 to 25 ml, from 2 to 20 ml, from 2 to 15 ml, from 2 to 10 ml , from 4 to 25 ml, from 4 to 20 ml, from 4 to 15 ml, from 4 to 10 ml, from 6 to 25 ml, from 6 to 20 ml, from 6 to 15 ml, from 6 to 10 ml, from 8 to 25 ml, from 8 to 20 ml, from 8 to 15 ml, from 10 to 25 ml, from 10 to 20 ml, from 10 to 15 ml, from 15 to 25 ml or from 15 to 20 ml.

The term "subject" is intended to include all animals shown to or expected to have microvesicles. Preferably, the subject is a mammal, a human or nonhuman primate, a dog, a cat, a horse, a cow, other farm animals, or a rodent (e.g. mice, rats, guinea pig. etc.). The term "subject" and "individual" are used interchangeably herein.

Methods of isolating microvesicles from a biological sample are known in the art. For example, a method of differential centrifugation is described in a paper by Raposo et al. (Raposo et al., 1996), and similar methods are detailed in the Examples section herein. Methods of anion exchange and/or gel permeation chromatography are described in US Patent Nos. 6,899,863 and 6,812,023. Methods of sucrose density gradients or organelle electrophoresis are described in U.S. Patent No. 7,198,923. A method of magnetic activated cell sorting (MACS) is described in (Taylor and Gercel-Taylor, 2008). A method of nanomembrane ultrafiltration concentrator is described in (Cheruvanky et al., 2007). Preferably, microvesicles can be identified and isolated from bodily fluid of a subject by a newly developed microchip technology that uses a unique microfluidic platform to efficiently and selectively separate tumor derived microvesicles. This technology, as described in a paper by Nagrath et al. (Nagrath et al., 2007), can be adapted to identify and separate microvesicles using similar principles of capture and separation as taught in the paper.

The microvesicles isolated from a bodily fluid may be enriched for those originating from a specific cell type, for example, lung, pancreas, stomach, intestine, bladder, kidney, ovary, testis, skin, colorectal, breast, prostate, brain, esophagus, liver, placenta, fetus cells. Because the microvesicles often carry surface molecules such as antigens from their donor cells, surface molecules may be used to identify, isolate and/or enrich for microvesicles from a specific donor cell type (Al-Nedawi et al., 2008; Taylor and Gercel-Taylor, 2008). In this way, microvesicles originating from distinct cell populations can be analyzed for their nucleic acid content. For example, tumor (malignant and non-malignant) microvesicles carry tumor-associated surface antigens and may be detected, isolated and/or enriched via these specific tumor-associated surface antigens. The surface antigen may be epithelial-cell-adhesion-molecule (EpCAM), which is specific to microvesicles from carcinomas of lung, colorectal, breast, prostate, head and neck, and hepatic origin, but not of hematological cell origin (Balzar et al., 1999; Went et al., 2004). In another example, the surface antigen is CD24, which is a glycoprotein specific to urine microvesicles (Keller et al., 2007). In yet another example, the surface antigen is selected from a group of molecules CD70, carcinoembryonic antigen (CEA), EGFR, EGFRvIII and other variants, Fas ligand, TRAIL, tranferrin receptor, p38.5, p97 and HSP72. Additionally, tumor specific microvesicles may be characterized by the lack of surface markers, such as CD80 and CD86.

The isolation of microvesicles from specific cell types can be accomplished, for example, by using antibodies, aptamers, aptamer analogs or molecularly imprinted polymers specific for a desired surface antigen. The surface antigen may be specific for a cancer type. The surface antigen may be specific for a cell type which is not necessarily cancerous. One example of a method of microvesicle separation based on cell surface antigen is provided in U.S. Patent No. 7,198,923. As described in, e.g., U.S. Patent Nos. 5,840,867 and 5,582,981, WO/2003/050290 and a publication by Johnson et al. (Johnson et al., 2008), aptamers and their analogs specifically bind surface molecules and can be used as a separation tool for retrieving cell type-specific microvesicles. Molecularly imprinted polymers also specifically recognize surface molecules as described in, e.g., US Patent Nos. 6,525,154, 7,332,553 and 7,384,589 and a publication by Bossi et al. (Bossi et al., 2007) and are a tool for retrieving and isolating cell type-specific microvesicles.

Preferably, the microvesicles are isolated from a bodily fluid sample using an affinity-based filter column. For example, the column contains agents or moieties that specifically bind to tumor-derived microvesicles, or microvesicles from a certain tissue (*i.e.,* diseased tissue, tumor tissue) or a specific cell type. For example, the column contains agents or moieties that specifically bind to tumor-associated antigens that are presented on the surface of the microvesicles, such that the microvesicles of interest are retained on the column, while other cells, debris, and non-specific microvesicles can be discarded. The bodily fluid samples may be pre-processed, by centrifugation or filtration, prior to utilizing the affinity-based filter column.

Alternatively, the microvesicles may be isolated by a combination of centrifugation, filtration, and/or concentration steps. For example, the bodily fluid samples may be first pre-processed by using a method comprising at least one filtration step. For example, a course filter (0.8 micron) is utilized to remove cells and cell debris. This filtration may be followed by an ultrafiltration step to remove solvent and small molecule analytes while retaining the microvesicles. The filters used in the initial filtration can be any size that is sufficient to remove cells and cell debris, for example, any size greater than 0.22 microns. To isolate the microvesicles, the pre-processed samples are then subjected to a filtration concentration step, wherein a filter that has a molecular weight cutoff is utilized to retain and concentrate the microvesicles that are greater than 10 nm in diameter. For example, the sample is then concentrated to a volume of less than 1 ml, preferably 100-200 ul. For example, the molecular weight cutoff is at least 100 kDa.

After isolation and concentration of the microvesicles, the samples are pretreated with an RNase inhibitor, prior to nucleic acid extraction, to prevent digestion of extracted RNA and enhance the quality of the extraction. Optionally, the samples may be washed at least once using the appropriate buffer to further enrich or purify the microvesicle fraction. The samples may be washed twice using the appropriate buffer to further enrich or purify the microvesicle fraction. Optionally, the concentrated microvesicles are lysed on the filter used in the pre-processing step prior to extraction of DNA and/or RNA.

Optionally, control particles may be added to the sample prior to microvesicle isolation or nucleic acid extraction to serve as an internal control to evaluate the efficiency or quality of microvesicle purification and/or nucleic acid extraction. These control particles include Q-beta bacteriophage, virus particles, or any other particle that contains control nucleic acids (e.g., at least one control target gene) that may be naturally occurring or engineered by recombinant DNA techniques. The quantity of control particles may be known before the addition to the sample. The control target gene can be quantified using real-time PCR analysis. Quantification of a control target gene can be used to determine the efficiency or quality of the microvesicle purification or nucleic acid extraction processes.

The methods described herein may include the use of a control particle to determine or evaluate the quality of the microvesicle isolation and/or microvesicle nucleic acid extraction. Control particles collectively refer to particles of the size range of microvesicles that are added at some point during the microvesicle isolation or nucleic acid extraction process, wherein the particles contain control nucleic acids, such as DNA or RNA. Specifically, the control nucleic acids comprise at least one target gene to be assayed or measured for determining the amount of recovery of the control particle during the isolation or extraction process.

Preferably, the control particle is a Q-beta bacteriophage, referred to herein as "Q-beta particle". The Q-beta particle used in the methods described herein may be a naturally-occurring virus particle or may be a recombinant or engineered virus, in which at least one component of the virus particle (e.g., a portion of the genome genome or coat protein) is synthesized by recombinant DNA or molecular biology techniques known in the art. Q-beta is a member of the leviviridae family, characterized by a linear, single-stranded RNA genome that consists of 3 genes encoding four viral proteins: a coat protein, a maturation protein, a lysis protein, and RNA replicase. Due to its similar size to average microvesicles, Q-beta can be easily purified from a biological sample using the same purification methods used to isolate microvesicles, as described herein. In addition, the low complexity of the Q-beta viral single-stranded gene structure is advantageous for its use as a control in amplification-based nucleic acid assays. The Q-beta particle contains a control target gene or control target sequence to be detected or measured for the quantification of the amount of Q-beta particle in a sample. For example, the control target gene is the Q-beta coat protein gene. After addition of the Q-beta particles to the urine sample or isolated urine-derived microvesicles, the nucleic acids from the Q-beta particle are extracted along with the nucleic acids from the microvesicles and/or urine sample using the extraction methods described herein. Detection of the Q-beta control target gene can be determined by RT-PCR analysis, for example, simultaneously with the biomarkers of interest (*i.e.,* BRAF). A standard curve of at least 2, 3, or 4 known concentrations in 10-fold dilution of a control target gene can be used to determine copy number. The copy number detected and the quantity of Q-beta particle added can be compared to determine the quality of the isolation and/or extraction process.

Preferably, the Q-beta particles are added to the urine sample prior to nucleic extraction. For example, the Q-beta particles are added to the urine sample prior to ultrafiltration and/or after the pre-filtration step.

50, 100, 150, 200, 250, 300, 350, 400, 450, 500, 1,000 or 5,000 copies of Q-beta particles may be added to a bodily fluid sample. Preferably, 100 copies of Q-beta particles are added to a bodily fluid sample. The copy number of Q-beta particles can be calculated based on the ability of the Q-beta bacteriophage to infect target cells. Thus, the copy number of Q-beta particles is correlated to the colony forming units of the Q-beta bacteriophage.

Following the isolation of microvesicles from a biological sample, nucleic acid may be extracted from the isolated or enriched microvesicle fraction. Nucleic acid molecules can be isolated from a microvesicle using any number of procedures, which are well-known in the art, the particular isolation procedure chosen being appropriate for the particular biological sample. The extracted nucleic acids can be DNA and/or RNA. The DNA may be extracted. RNA may be extracted. Both DNA and RNA may be extracted. The RNA can be messenger RNA, transfer RNA, ribosomal RNA, small RNAs, non-coding RNAs. Additional steps may be performed during the nucleic extraction process to improve or enhance the quality of the extracted nucleic acids. Such additional steps are described in WO2011/009104.

High quality RNA extractions are highly desirable because RNA degradation can seriously affect downstream assessment of the extracted RNA, such as in gene expression and mRNA analysis, as well as analysis of non-coding RNA such as small RNA and microRNA. The novel methods described herein enable one to extract high quality nucleic acids from a biological sample such as microvesicles so that an accurate analysis of gene expression and mutational level within the exosomes can be carried out. For example, when increased concentrations of protease (5X, 10X) or RNase inhibitors are used as an extraction enhancing agent, the amount and integrity of RNA isolated from urinary microvesicles is increased significantly.

The extracted nucleic acid may be RNA. RNAs are then preferably reverse-transcribed into complementary DNAs before further amplification. Such reverse transcription may be performed alone or in combination with an amplification step. One example of a method combining reverse transcription and amplification steps is reverse transcription polymerase chain reaction (RT-PCR), which may be further modified to be quantitative, e.g., quantitative RT-PCR as described in US Patent No. 5,639,606.

Nucleic acid amplification methods include, without limitation, polymerase chain reaction (PCR) (US Patent No. 5,219,727) and its variants such as in situ polymerase chain reaction (US Patent No. 5,538,871), quantitative polymerase chain reaction (US Patent No. 5,219,727), nested polymerase chain reaction (US Patent No. 5,556,773), self sustained sequence replication and its variants (Guatelli et al., 1990), transcriptional amplification system and its variants (Kwoh et al., 1989), Qb Replicase and its variants (Miele et al., 1983), cold-PCR (Li et al., 2008) or any other nucleic acid amplification methods, followed by the detection of the amplified molecules using techniques well known to those of skill in the art. Especially useful are those detection schemes designed for the detection of nucleic acid molecules if such molecules are present in very low numbers. The analysis of nucleic acids present in the microvesicles is quantitative and/or qualitative. For quantitative analysis, the amounts (expression levels), either relative or absolute, of specific nucleic acids of interest within the microvesicles are measured with methods known in the art (described below). For qualitative analysis, the species of specific nucleic acids of interest within the microvesicles, whether wild type or variants, are identified with methods known in the art (described below).

"Genetic aberrations" is used herein to refer to the nucleic acid amounts as well as nucleic acid variants within the microvesicles. Specifically, genetic aberrations include, without limitation, over-expression of a gene (e.g., oncogenes) or a panel of genes, under-expression of a gene (e.g., tumor suppressor genes such as p53 or RB) or a panel of genes, alternative production of splice variants of a gene or a panel of genes, gene copy number variants (CNV) (e.g. DNA double minutes) (Hahn, 1993), nucleic acid modifications (e.g., methylation, acetylation and phosphorylations), single nucleotide polymorphisms (SNPs), chromosomal rearrangements (e.g., inversions, deletions and duplications), and mutations (insertions, deletions, duplications, missense, nonsense, synonymous or any other nucleotide changes) of a gene or a panel of genes, which mutations, in many cases, ultimately affect the activity and function of the gene products, lead to alternative transcriptional splicing variants and/or changes of gene expression level.

The determination of such genetic aberrations can be performed by a variety of techniques known to the skilled practitioner. For example, expression levels of nucleic acids, alternative splicing variants, chromosome rearrangement and gene copy numbers can be determined by microarray analysis (US Patent Nos. 6,913,879, 7,364,848, 7,378,245, 6,893,837 and 6,004,755) and quantitative PCR. Particularly, copy number changes may be detected with the Illumina Infinium II whole genome genotyping assay or Agilent Human Genome CGH Microarray (Steemers et al., 2006). Nucleic acid modifications can be assayed by methods described in, e.g., US Patent No. 7,186,512 and patent publication WO/2003/023065. Particularly, methylation profiles may be determined by Illumina DNA Methylation OMA003 Cancer Panel. SNPs and mutations can be detected by hybridization with allele- specific probes, enzymatic mutation detection, chemical cleavage of mismatched heteroduplex (Cotton et al., 1988), ribonuclease cleavage of mismatched bases (Myers et al., 1985), mass spectrometry (US Patent Nos. 6,994,960, 7,074,563, and 7,198,893), nucleic acid sequencing, single strand conformation polymorphism (SSCP) (Orita et al., 1989), denaturing gradient gel electrophoresis (DGGE) (Fischer and Lerman, 1979a; Fischer and Lerman, 1979b), temperature gradient gel electrophoresis (TGGE) (Fischer and Lerman, 1979a; Fischer and Lerman, 1979b), restriction fragment length polymorphisms (RFLP) (Kan and Dozy, 1978a; Kan and Dozy, 1978b), oligonucleotide ligation assay (OLA), allele- specific PCR (ASPCR) (US Patent No. 5,639,611), ligation chain reaction (LCR) and its variants (Abravaya et al., 1995; Landegren et al., 1988; Nakazawa et al., 1994), flow-cytometric heteroduplex analysis (WO/2006/113590) and combinations/modifications thereof. Notably, gene expression levels may be determined by the serial analysis of gene expression (SAGE) technique (Velculescu et al., 1995). In general, the methods for analyzing genetic aberrations are reported in numerous publications, not limited to those cited herein, and are available to skilled practitioners. The appropriate method of analysis will depend upon the specific goals of the analysis, the condition/history of the patient, and the specific cancer(s), diseases or other medical conditions to be detected, monitored or treated.

Mutations of a gene which is associated with a disease such as cancer (e.g. via nucleotide variants, over-expression or under-expression) may be detected by analysis of nucleic acids in micro vesicles, which nucleic acids are derived from the genome itself in the cell of origin or exogenous genes introduced through viruses. The nucleic acid sequences may be complete or partial, as both are expected to yield useful information in diagnosis and prognosis of a disease. The sequences may be sense or anti-sense to the actual gene or transcribed sequences. The skilled practitioner will be able to devise detection methods for a nucleotide variance from either the sense or anti-sense nucleic acids which may be present in a microvesicle. Many such methods involve the use of probes which are specific for the nucleotide sequences which directly flank, or contain the nucleotide variances. Such probes can be designed by the skilled practitioner given the knowledge of the gene sequences and the location of the nucleic acid variants within the gene. Such probes can be used to isolate, amplify, and/or actually hybridize to detect the nucleic acid variants, as described in the art and herein.

Determining the presence or absence of a particular nucleotide variant or plurality of variants in the nucleic acid within microvesicles from a subject can be performed in a variety of ways. A variety of methods are available for such analysis, including, but not limited to, PCR, hybridization with allele- specific probes, enzymatic mutation detection, chemical cleavage of mismatches, mass spectrometry or DNA sequencing, including minisequencing. Hybridization with allele specific probes can be conducted in two formats: 1) allele specific oligonucleotides bound to a solid phase (glass, silicon, nylon membranes) and the labeled sample in solution, as in many DNA chip applications, or 2) bound sample (often cloned DNA or PCR amplified DNA) and labeled oligonucleotides in solution (either allele specific or short so as to allow sequencing by hybridization). Diagnostic tests may involve a panel of variances, often on a solid support, which enables the simultaneous determination of more than one variance. Determining the presence of at least one nucleic acid variance in the microvesicle nucleic acid may entail a haplotyping test. Methods of determining haplotypes are known to those of skill in the art, as for example, in WO 00/04194.

The determination of the presence or absence of a nucleic acid variant(s) may involve determining the sequence of the variant site or sites (the exact location within the sequence where the nucleic acid variation from the norm occurs) by methods such as polymerase chain reaction (PCR), chain terminating DNA sequencing (US Patent No. 5547859), minisequencing (Fiorentino et al., 2003), oligonucleotide hybridization, pyrosequencing, Illumina genome analyzer, deep sequencing, mass spectrometry or other nucleic acid sequence detection methods. Methods for detecting nucleic acid variants are well known in the art and disclosed in WO 00/04194. In an exemplary method, the diagnostic test comprises amplifying a segment of DNA or RNA (generally after converting the RNA to complementary DNA) spanning one or more known variants in the desired gene sequence. This amplified segment is then sequenced and/or subjected to electrophoresis in order to identify nucleotide variants in the amplified segment.

The present description provides a method of screening for nucleotide variants in the nucleic acid of microvesicles isolated as described herein. This can be achieved, for example, by PCR or, alternatively, in a ligation chain reaction (LCR) (Abravaya et al., 1995; Landegren et al., 1988; Nakazawa et al., 1994). LCR can be particularly useful for detecting point mutations in a gene of interest (Abravaya et al., 1995). The LCR method comprises the steps of designing degenerate primers for amplifying the target sequence, the primers corresponding to one or more conserved regions of the nucleic acid corresponding to the gene of interest, amplifying PCR products with the primers using, as a template, a nucleic acid obtained from a micro vesicle, and analyzing the PCR products. Comparison of the PCR products of the microvesicle nucleic acid to a control sample (either having the nucleotide variant or not) indicates variants in the microvesicle nucleic acid. The change can be either an absence or presence of a nucleotide variant in the microvesicle nucleic acid, depending upon the control.

Analysis of amplification products can be performed using any method capable of separating the amplification products according to their size, including automated and manual gel electrophoresis, mass spectrometry, and the like.

Alternatively, the amplification products can be analyzed based on sequence differences, using SSCP, DGGE, TGGE, chemical cleavage, OLA, restriction fragment length polymorphisms as well as hybridization, for example, nucleic acid microarrays.

The methods of nucleic acid isolation, amplification and analysis are routine for one skilled in the art and examples of protocols can be found, for example, in Molecular Cloning: A Laboratory Manual (3-Volume Set) Ed. Joseph Sambrook, David W. Russel, and Joe Sambrook, Cold Spring Harbor Laboratory, 3rd edition (January 15, 2001), ISBN: 0879695773. A particular useful protocol source for methods used in PCR amplification is PCR Basics: From Background to Bench by Springer Verlag; 1st edition (October 15, 2000), ISBN: 0387916008.

Many methods of diagnosis performed on a tumor biopsy sample can be performed with microvesicles since tumor cells, as well as some normal cells are known to shed microvesicles into bodily fluid and the genetic aberrations within these microvesicles reflect those within tumor cells as demonstrated herein. Furthermore, methods of diagnosis using microvesicles have characteristics that are absent in methods of diagnosis performed directly on a tumor biopsy sample. For example, one particular advantage of the analysis of microvesicular nucleic acids, as opposed to other forms of sampling of tumor/cancer nucleic acid, is the availability for analysis of tumor/cancer nucleic acids derived from all foci of a tumor or genetically heterogeneous tumors present in an individual. Biopsy samples are limited in that they provide information only about the specific focus of the tumor from which the biopsy is obtained. Different tumorous/cancerous foci found within the body, or even within a single tumor often have different genetic profiles and are not analyzed in a standard biopsy. However, analysis of the microvesicular nucleic acids from an individual presumably provides a sampling of all foci within an individual. This provides valuable information with respect to recommended treatments, treatment effectiveness, disease prognosis, and analysis of disease recurrence, which cannot be provided by a simple biopsy.

Identification of genetic aberrations associated with specific diseases and/or medical conditions by the methods described herein can also be used for prognosis and treatment decisions of an individual diagnosed with a disease or other medical condition such as cancer. Identification of the genetic basis of a disease and/or medical condition provides useful information guiding the treatment of the disease and/or medical condition. For example, many forms of chemotherapy have been shown to be more effective on cancers with specific genetic abnormalities/aberrations. One example is the use of BRAF inhibitors for treating cancers with BRAF activating mutations. It may be useful to use combination therapy, wherein the combination therapy comprises a BRAF inhibitor and another chemotherapeutic agent, drug, surgery or radiation therapy.

Genetic aberrations in other genes have also been found to influence the effectiveness of treatments. As disclosed in the publication by Furnari et al. (Furnari et al., 2007), mutations in a variety of genes affect the effectiveness of specific medicines used in chemotherapy for treating brain tumors. The identification of these genetic aberrations in the nucleic acids within microvesicles will guide the selection of proper treatment plans.

As such, the present description relates to a method for monitoring disease (e.g. cancer) progression in a subject, and also to a method for monitoring disease recurrence in an individual. These methods comprise the steps of isolating microvesicles from a bodily fluid of an individual, as discussed herein, and analyzing nucleic acid within the microvesicles as discussed herein (e.g. to create a genetic profile of the microvesicles). The presence/absence of a certain genetic aberration/profile is used to indicate the presence/absence of the disease (e.g. cancer) in the subject as discussed herein. The process is performed periodically over time, and the results reviewed, to monitor the progression or regression of the disease, or to determine recurrence of the disease. Put another way, a change in the genetic profile indicates a change in the disease state in the subject. The period of time to elapse between sampling of microvesicles from the subject, for performance of the isolation and analysis of the microvesicle, will depend upon the circumstances of the subject, and is to be determined by the skilled practitioner. Such a method would prove extremely beneficial when analyzing a nucleic acid from a gene that is associated with the therapy undergone by the subject. For example, a gene which is targeted by the therapy can be monitored for the development of mutations which make it resistant to the therapy, upon which time the therapy can be modified accordingly. The monitored gene may also be one which indicates specific responsiveness to a specific therapy.

Aspects of the present description also relate to the fact that a variety of non-cancer diseases and/or medical conditions also have genetic links and/or causes, and such diseases and/or medical conditions can likewise be diagnosed and/or monitored by the methods described herein. Many such diseases are metabolic, infectious or degenerative in nature.

Selection of an individual from whom the microvesicles are isolated is performed by the skilled practitioner based upon analysis of one or more of a variety of factors. Such factors for consideration are whether the subject has a family history of a specific disease (e.g. a cancer), has a genetic predisposition for such a disease, has an increased risk for such a disease due to family history, genetic predisposition, other disease or physical symptoms which indicate a predisposition, or environmental reasons. Environmental reasons include lifestyle, exposure to agents which cause or contribute to the disease such as in the air, land, water or diet. In addition, having previously had the disease, being currently diagnosed with the disease prior to therapy or after therapy, being currently treated for the disease (undergoing therapy), being in remission or recovery from the disease, are other reasons to select an individual for performing the methods.

The methods described herein are optionally performed with the additional step of selecting a gene or nucleic acid for analysis, prior to the analysis step. This selection can be based on any predispositions of the subject, or any previous exposures or diagnosis, or therapeutic treatments experienced or concurrently undergone by the subject.

The cancer diagnosed, monitored or otherwise profiled, can be any kind of cancer. This includes, without limitation, epithelial cell cancers such as lung, ovarian, cervical, endometrial, breast, brain, colon and prostate cancers. Also included are gastrointestinal cancer, head and neck cancer, non- small cell lung cancer, cancer of the nervous system, kidney cancer, retina cancer, skin cancer, liver cancer, pancreatic cancer, genital-urinary cancer and bladder cancer, melanoma, and leukemia. In addition, the methods and compositions are equally applicable to detection, diagnosis and prognosis of non-malignant tumors in an individual (e.g. neurofibromas, meningiomas and schwannomas). Preferably, the cancer is associated with oncogenic or activating mutantions of BRAF.

### EXAMPLES

### Example 1: Preparation of nucleic acids from microvesicles

The instant disclosure provides methods for extraction of nucleic acids from microvesicles isolated from patient samples. Specifically, DNA and RNA were extracted from a melanoma patient plasma sample. Prior to extraction, the samples were preprocessed. For example, a plasma sample within the range of 2-20 ml was spun at 120,000 x g for 80 minutes in an ultracentrifuge. Optionally, RNase inhibitors, such as RNasin Plus (40 u/µl, Promega) or Superasin (20 u/µl, Ambion), are added to the pellet and incubated for 5 minutes at room temperature. The microvesicle-containing pellet was then processed for either DNA extraction using the Qiagen DNeasy Blood and Tissue Kit (Cat. No. 69504) or RNA extraction using Qiagen miRNeasy Kit (Cat. No. 217004) using the manufacturer's recommended protocol.

It may be preferable for the extracted RNA to be reverse transcribed into cDNA before analysis. The RNA is reverse transcribed, using commercially available cDNA synthesis kits that contain reverse transcriptase, such as Superscript ® VILO™ (Invitrogen). The reverse transcription reaction was prepared as follows:

| | (µl) × 1 | × 4.4 |
|---|---|---|
| 5X VILO™ Reaction Mix | 4 | 17.6 |
| 10X SuperScript® Enzyme Mix | 2 | 8.8 |
| RNA (up to 2.5 µg) | 12 | - |
| Nuclease free water Total volume | 2 20 | 8.8 |

The cDNA synthesis reaction is then run on a thermocycler, such as the Vert PCR machine. The cDNA reaction program are as follows:

| | |
|---|---|
| 1. 25°C | 10 min |
| 2. 42°C | 70 min |
| 3. 85°C | 5 min |
| 4. 4°C | |

The cDNA is then frozen at -20°C or -80°C for long term storage.

### Example 2: Detection of BRAF mutations using a QPCR approach

Plasma samples from melanoma patients were analyzed using the methods disclosed herein. The biopsy of the original tumor revealed a V600E BRAF mutation in both sample 081 and MGHSS04. Sample 055 and 091 was from melanoma patients who werewas negative for V600E BRAF mutation.

First, a microvesicle fraction was obtained from the plasma and DNA or RNA was extracted from the microvesicles utilizing the methods disclosed herein. For the QPCR assay, 5 µl of the extracted DNA or 2 µl cDNA was analyzed using the following QPCR primers and probe:
BRAF WT forward: AAAAATAGGTGATTTTGGTCTAGCTACAGT (SEQ ID NO: 3)
BRAF MT ARMS forward: AAAAATAGGTGATTTTGGTCTAGCTACATA (SEQ ID NO: 4)
BRAF JS E15 Reverse: TGGATCCAGACAACTGTTCAA (SEQ ID NO: 5)
BRAF AZ E15 probe (VIC-MGB): GATGGAGTGGGTCCCATCAG (SEQ ID NO: 6)

The QPCR reaction was prepared using Taqman Gen Expression Master Mix (Applied Biosystems 4369016) as follows:

| | |
|---|---|
| BRAF WT forward or MT ARMS forward (18 µM) | 1 µl |
| BRAF JS E15 reverse (18 µM) | 1 µl |
| BRAF E15 VIC probe (5 µM) | 1 µl |
| 2x Taqman Gene Expression Master Mix | 10 µl |
| Extracted DNA or cDNA | 5 µl or 2 µl |
| H2O | Add to 20 µl |
| Total | 20 µl |

The amplification program used was as follows:
1. 50°C for 2 minutes
2. 95°C for 10 minutes
3. 95°C for 15 seconds
4. 60°C for 60 seconds
5. Repeat steps 3 and 4 for 50 total cycles

Each sample was analyzed by QPCR in triplicate. For sample 081, the Ct values were 34.7, 34.79, and 36.89. For sample MGHSS04, the Ct values were 35.28, 34.69, and 35.21. Amplification threshold was manually set at above baseline. The amplification plot for samples 055 and 081 are shown in Figure 1. The amplification plot for sample MGHSS04 is shown in Figure 2. The amplification plot for sample 091 is shown in Figure 3.

As shown in Figures 1, 2 and 3, the mutant form of BRAF was detected in sample 081 and sample MGHSS04, as expected, in accordance with the biopsy data from the tumor tissue. Accordingly, a mutant form of BRAF was not detected in sample 055 or sample 091, as expected. These results demonstrate that mutant BRAF can be accurately and reproducibly detected from nucleic acids extracted from biological samples by QPCR assay.

### Example 3: Use of DNA and RNA analysis to enhance detection sensitivity

A panel of melanoma patients was analyzed by QPCR for BRAF mutation status. All patients were positive for BRAF mutations by biopsy analysis. Specifically, biopsy samples from patient 051, 057, 061, 074, 085, 089, 090, 098, 107, and 109 contained V600E BRAF mutations. The biopsy sample from patient 062 exhibited the V600K BRAF mutation.

DNA and RNA were extracted from plasma and serum samples from the panel of melanoma patients by the methods disclosed herein. The nucleic acids were subjected to QPCR analysis for detection of BRAF mutations. Results of the QPCR analysis are summarized in Table 1. For all samples, analysis of either DNA or RNA resulted in accurate detection of the presence of the BRAF mutation. For some samples, detection of both allowed for increased sensitivity of detection of the BRAF mutation, and shows that detection of BRAF from microvesicle-extracted nucleic acids could be valuable for diagnosis and prognosis of cancer.

**Table 1. Summary of results from QPCR analysis from DNA and RNA samples from a panel of melanoma patients.**

| **Sample ID** | **Biopsy BRAF mutant** | **QPCR result on DNA** | **QPCR result on RNA** |
|---|---|---|---|
| 051 | V600E | + | - |
| 057 | V600E | + | - |
| 062 | V600K | + | - |
| 085 | V600E | + | - |
| 089 | V600E | + | - |
| 074 | V600E | - | + |
| 098 | V600E | - | + |
| 109 | V600E | - | + |
| 061 | V600E | + | + |
| 090 | V600E | + | + |
| 107 | V600E | + | + |

### References

Abravaya, K., J.J. Carrino, S. Muldoon, and H.H. Lee. 1995 Detection of point mutations with a modified ligase chain reaction (Gap-LCR). Nucleic Acids Res. 23:675-82.
Al-Nedawi, K., B. Meehan, J. Micallef, V. Lhotak, L. May, A. Guha, and J. Rak. 2008. Intercellular transfer of the oncogenic receptor EGFRvIII by microvesicles derived from tumour cells. Nat Cell Biol. 10:619-24.
Balzar, M., M.J. Winter, C.J. de Boer, and S.V. Litvinov. 1999. The biology of the 17-1A antigen (Ep-CAM). J Mol Med. 77:699-712.
Bossi, A., F. Bonini, A.P. Turner, and S.A. Piletsky. 2007. Molecularly imprinted polymers for the recognition of proteins: the state of the art. Biosens Bioelectron. 22:1131-7.
Cheruvanky, A., H. Zhou, T. Pisitkun, J.B. Kopp, M.A. Knepper, P.S. Yuen, and R.A. Star. 2007. Rapid isolation of urinary exosomal biomarkers using a nanomembrane ultrafiltration concentrator. Am J Physiol Renal Physiol. 292:F1657-61.
Cotton, R.G., N.R. Rodrigues, and R.D. Campbell. 1988. Reactivity of cytosine and thymine in single-base-pair mismatches with hydroxylamine and osmium tetroxide and its application to the study of mutations. Proc Natl Acad Sci U S A. 85:4397-401.
Davies H, Bignell GR, Cox C, Stephens P, Edkins S, Clegg S, Teague J, Woffendin H, Garnett MJ, Bottomley W, Davis N, Dicks E, Ewing R, Floyd Y, Gray K, Hall S, Hawes R, Hughes J, Kosmidou V, Menzies A, Mould C, Parker A, Stevens C, Watt S, Hooper S, Wilson R, Jayatilake H, Gusterson BA, Cooper C, Shipley J, Hargrave D, Pritchard-Jones K, Maitland N, Chenevix-Trench G, Riggins GJ, Bigner DD, Palmieri G, Cossu A, Flanagan A, Nicholson A, Ho JW, Leung SY, Yuen ST, Weber BL, Seigler HF, Darrow TL, Paterson H, Marais R, Marshall CJ, Wooster R, Stratton MR, Futreal PA. 2002. Mutations of the BRAF gene in human cancer. Nature 417(6892):949-54.
Diehl, F., K. Schmidt, M.A., Choti, K. Romans, S. Goodman, M. Li, K. Thornton, N. Agrawal, L. Sokoll, S.A. Szabo, K.W. Kintzler, B. Vogelstein, and L.A. Diaz, Jr. 2008. Circulating mutant DNA to assess tumor dynamics. Nat Med. 13:985-90.
Fiorentino, F., M.c. Magli, D. Podini, Ap.P. Ferraretti, A. Nuccitelli, N. Vitale, M. Baldi, and L. Bianaroli. 2003. The minisequencing mehdo: an alternative strategy for preimplantation genetic diagnosis of single gene disorders. Mol Hum Reprod. 9:399-410.
Fischer, S.G., and L.S. Lerman 1979a. Length -independent separation of DNA restriction fragments in two-dimentional gel electrophoresis. Cell. 16:191-200.
Fischer, S.G., and L.S. Lerman 1979b. Two-dimentional electrophoretic separation of restriction enzyme fragments of DNA. Methods Enzymol. 68:183-91.
Furnari, F.B., T. Fenton, R.M. Bachoo, A. Mukasa, J.M. Stommel, A. Stegh, W.C. Hahn, K.L. Ligon, D.N. Louis, C. Brennan, L. Chin, R.A. DePinho, and W.K. Cavenee. 2007. Malignant astrocytic glioma: genetics, biology, and paths to treatment. Genes Dev. 21:2683-710.
Gormally, E., E. Caboux, P. vineis, and P. Hainaut. 2007. Circulating free DNA in plasma or serum as biomarker of carcinogenesis: practical aspects and biological significance. Mutat Res. 635:105-17.
Guatelli, J.C., K.M. Whitfield, D.Y. Kwoh, K.J. Barringer, D.D. Richman, and T.R. Gingeras. 1990. Isothermal, in vitro amplification of nucleic acids by a multienzyme reaction modeled after retroviral replication. Proc Natl Acad Sci U S A. 87:1874-8.
Hahn, P.J. 1993. Molecular biology of double-minute chromosomes. Bioessays. 15:477-84.
Johnson, S., D. Evans, S. Laurenson, D. Paul, A.G. Davies, P.K. Ferrigno, and C. Walti. 2008. Surface-immobilized peptide aptamers as probe molecules for protein detection. Anal Chem. 80:978-83.
Keller, S., C. Rupp, A. Stoeck, S. Runz, M. Fogel, S. Lugert, H.D. Hager, M.S. Abdel-Bakky, P. Gutwein, and P. Altevogt. 2007. CD24 is a marker of exosomes secreted into urine and amniotic fluid. Kidney Int. 72:1095-102.
Kwoh, D.Y., G.R. Davis, K.M. Whitfield, H.L. Chappelle, L.J. DiMichele, and T.R. Gingeras. 1989. Transcription-based amplification system and detection of amplified human immunodeficiency virus type 1 with a bead-based sandwich hybridization format. Proc Natl Acad Sci USA. 86:1173-7.
Landegren, U., R. Kaiser, J. Sanders, and L. Hood. 1988. A ligase-mediated gene detection technique. Science. 241:1077-80.
Li, J., L. Wang, H. Mamon, M.H. Kulke, R. Berbeco, and G.M. Makrigiorgos. 2008. Replacing PCR with COLD-PCR enriches variant DNA sequences and redefines the sensitivity of genetic testing. Nat Med. 14:579-84.
Miele, E.A., D.R. Mills, and F.R. Kramer. 1983. Autocatalytic replication of a recombinant RNA. J Mol Biol. 171:281-95.
Myers, R.M., Z. Larin, and T. Maniatis. 1985. Detection of single base substitutions by ribonuclease cleavage at mismatches in RNA:DNA duplexes. Science. 230:1242-6.
Nagrath, S., L.V. Sequist, S. Maheswaran, D.W. Bell, D. Irimia, L. Ulkus, M.R. Smith, E.L. Kwak, S. Digumarthy, A. Muzikansky, P. Ryan, U.J. Balis, R.G. Tompkins, D.A. Haber, and M. Toner. 2007. Isolation of rare circulating tumour cells in cancer patients by microchip technology. Nature. 450:1235-9.
Nakazawa, H. D. English, P.L. randell, K. Nakazawa, N. Martel, B.K. Armstrong, and H. Yamasaki. 1994. UV and skin cancer: specific p53 gene mutation in normal skin as a biologically relevant exposure measurement. Proc Natl Acad Sci USA. 91:360-4.
Orita, M., H. Iwahana, H. Kanazawa, K. Hayashi, and T. Sekiya. 1989. Detection of polymorphisms of human DNA by gel electrophoresis as single-strand conformation polymorphisms. Proc Natl Acad Sci USA. 86:2766-70.
Raposo, G., H.W. Nijman, W. Stoorvogel, R. Liejendekker, C.V. Harding, C.J. Melief, and H.J. Geuze. 1996. B lymphocytes secrete antigen-presenting vesicles. J Exp Med. 183:1161-72.
Skog, J., T. Wurdinger, S. van Rijn, D.H. Meijer, L. Gainche, M. Sena-Esteves, W.T. Curry, Jr., B.S. Carter, A.M. Krichevsky, and X.O. Breakefield. 2008. Glioblastoma microvesicles transport RNA and proteins that promote tumour growth and provide diagnostic biomarkers. Nat Cell Biol. 10:1470-6.
Steemers, F.J., W. Chang, G. Lee, D.L. Barker, R. Shen, and K.L. Gunderson. 2006. Whole-genome genotyping with the single-base extension assay. Nat Methods. 3:31-3.
Taylor, D.D., and C. Gercel-Taylor. 2008. MicroRNA signatures of tumor-derived exosomes as diagnostic biomarkers of ovarian cancer. Gynecol Oncol. 110:13-21.
Van Dijk, E.L., G. Schilders, and G.J. Pruijn. 2007. Human cell growth requires a functional cytoplasmic exosome, which is involved in various mRNA decay pathways. RNA. 13:1027-35.
Went, P.T., A. Lugli, S. Meier, M. Bundi, M. Mirlacher, G. Sauter, and S. Dirnhofer. 2004. Frequent EpCam protein expression in human carcinomas. Hum Pathol. 35:122-8.

## Claims

1. A method for diagnosing cancer in a subject comprising:
a). isolating a microvesicle fraction from a biological sample from the subject;
b) extracting DNA and RNA from the microvesicles; and
c) detecting the presence or absence of a BRAF mutation in the extracted DNA and RNA,
wherein the presence of the BRAF mutation in the extracted DNA and RNA indicates the presence of cancer in the subject or a higher predisposition of the subject to develop cancer.

2. A method for determining a therapeutic regimen for treatment of a subject suffering from cancer comprising:
a) isolating a microvesicle fraction from a biological sample from the subject;
b) extracting DNA and RNA from the microvesicles; and
c) detecting the presence or absence of a BRAF mutation in the extracted DNA and RNA,
wherein the presence of the BRAF mutation in the extracted DNA and RNA indicates the use of a therapeutic regimen that comprises at least one kinase inhibitor.

3. The method of claim 1 or claim 2, wherein the cancer is melanoma, thyroid cancer, colorectal cancer, ovarian cancer, breast cancer, lung cancer, brain cancer, pancreas cancer, lymphoma or leukemia.

4. The method of claim 1 or 2, wherein the BRAF mutation is an activating mutation.

5. The method of claim 1 or 2, wherein the BRAF mutation encodes a mutant BRAF polypeptide wherein the mutant BRAF polypeptide is V600E.

6. The method of claim 1 or 2, wherein the BRAF mutation is T1799A.

7. The method of claim 2, wherein the kinase inhibitor is a RAF inhibitor or a MEK inhibitor.

8. The method of claim 7, wherein the RAF inhibitor is a BRAF-specific inhibitor.

9. The method of claim 1 or 2, wherein the biological sample is a bodily fluid sample.

10. The method of claim 9, wherein the bodily fluid sample is plasma, serum, cerebrospinal fluid, ascites fluid, bronchoalveolar lavage, and cyst fluid.

11. The method of claim 9, wherein the bodily fluid sample is in the range of 2-20 ml.

12. The method of claim 2, wherein the therapeutic regimen comprises a RAF inhibitor or a MEK inhibitor.

13. The method of claim 2, wherein the therapeutic regimen comprises vemurafenib or dabrafenib.

## Patentansprüche

1. Verfahren zur Diagnose von Krebs bei einem Patienten, umfassend:
a) Isolieren einer Mikrovesikelfraktion aus einer biologischen Probe von dem Patienten;
b) Extrahieren von DNA und RNA aus den Mikrovesikeln; und
c) Nachweisen der An- oder Abwesenheit einer BRAF-Mutation in der extrahierten DNA und RNA,
wobei die Anwesenheit der BRAF-Mutation in der extrahierten DNA und RNA auf die Anwesenheit von Krebs bei dem Patienten oder eine höhere Prädisposition des Patienten zum Entstehen von Krebs hinweist.

2. Verfahren zur Festlegung eines therapeutischen Regimes zur Behandlung eines an Krebs leidenden Patienten, umfassend:
a) Isolieren einer Mikrovesikelfraktion aus einer biologischen Probe von dem Patienten;
b) Extrahieren von DNA und RNA aus den Mikrovesikeln; und
c) Nachweisen der An- oder Abwesenheit einer BRAF-Mutation in der extrahierten DNA und RNA,
wobei die Anwesenheit der BRAF-Mutation in der extrahierten DNA und RNA auf die Verwendung eines therapeutischen Regimes hinweist, das mindestens einen Kinaseinhibitor umfasst.

3. Verfahren nach Anspruch 1 oder Anspruch 2, wobei der Krebs ein Melanom, ein Schilddrüsenkrebs, ein Kolorektalkrebs, ein Ovarialkrebs, ein Brustkrebs, ein Lungenkrebs, ein Hirnkrebs, ein Pankreaskrebs, ein Lymphom oder eine Leukämie ist.

4. Verfahren nach Anspruch 1 oder 2, wobei die BRAF-Mutation eine aktivierende Mutation ist.

5. Verfahren nach Anspruch 1 oder 2, wobei die BRAF-Mutation für ein mutiertes BRAF-Polypeptid kodiert, wobei das mutierte BRAF-Polypeptid V600E ist.

6. Verfahren nach Anspruch 1 oder 2, wobei die BRAF-Mutation T1799A ist.

7. Verfahren nach Anspruch 2, wobei der Kinaseinhibitor ein RAF-Inhibitor oder ein MEK-Inhibitor ist.

8. Verfahren nach Anspruch 7, wobei der RAF-Inhibitor ein BRAF-spezifischer Inhibitor ist.

9. Verfahren nach Anspruch 1 oder 2, wobei die biologische Probe eine Körperflüssigkeitsprobe ist.

10. Verfahren nach Anspruch 9, wobei die Körperflüssigkeitsprobe Plasma, Serum, Cerobrospinalflüssigkeit, Ascitesflüssigkeit, durch bronchoalveoläre Lavage gewonnene Flüssigkeit und Zystenflüssigkeit ist.

11. Verfahren nach Anspruch 9, wobei die Körperflüssigkeitsprobe im Bereich von 2 ml bis 20 ml liegt.

12. Verfahren nach Anspruch 2, wobei das therapeutische Regime einen RAF-Inhibitor oder einen MEK-Inhibitor umfasst.

13. Verfahren nach Anspruch 2, wobei das therapeutische Regime Vemurafenib oder Dabrafenib umfasst.

## Revendications

1. Procédé de diagnostic d'un cancer chez un sujet, comprenant :
a) l'isolement d'une fraction microvésiculaire à partir d'un échantillon biologique provenant du sujet ;
b) l'extraction de l'ADN et de l'ARN à partir des microvésicules ; et
c) la détection de la présence ou de l'absence d'une mutation de BRAF dans l'ADN et l'ARN extraits,
où la présence de la mutation de BRAF dans l'ADN et l'ARN extraits indique la présence d'un cancer chez le sujet ou une prédisposition plus élevée du sujet à développer un cancer.

2. Procédé d'élaboration d'un schéma thérapeutique pour le traitement d'un sujet souffrant d'un cancer, comprenant :
a) l'isolement d'une fraction microvésiculaire à partir d'un échantillon biologique provenant du sujet ;
b) l'extraction de l'ADN et de l'ARN à partir des microvésicules ; et
c) la détection de la présence ou de l'absence d'une mutation de BRAF dans l'ADN et l'ARN extraits,
où la présence de la mutation de BRAF dans l'ADN et l'ARN extraits indique l'utilisation d'un schéma thérapeutique qui comprend au moins un inhibiteur de kinase.

3. Procédé de la revendication 1 ou de la revendication 2, où le cancer est un mélanome, un cancer de la thyroïde, un cancer colorectal, un cancer de l'ovaire, un cancer du sein, un cancer du poumon, un cancer du cerveau, un cancer du pancréas, un lymphome ou une leucémie.

4. Procédé de la revendication 1 ou 2, où la mutation de BRAF est une mutation activatrice.

5. Procédé de la revendication 1 ou 2 où la mutation de BRAF code pour un polypeptide BRAF mutant, où le polypeptide BRAF mutant est le mutant V600E.

6. Procédé de la revendication 1 ou 2, où la mutation de BRAF est la mutation T1799A.

7. Procédé de la revendication 2, où l'inhibiteur de kinase est un inhibiteur de RAF ou un inhibiteur de MEK.

8. Procédé de la revendication 7, où l'inhibiteur de RAF est un inhibiteur spécifique de BRAF.

9. Procédé de la revendication 1 ou 2, où l'échantillon biologique est un échantillon d'un liquide corporel.

10. Procédé de la revendication 9, où l'échantillon de liquide corporel est un échantillon de plasma, de sérum, de liquide céphalorachidien, d'un liquide d'ascite, d'un lavage bronchoalvéolaire et d'un liquide kystique.

11. Procédé de la revendication 9, où l'échantillon de liquide corporel est dans la plage de 2 - 20 ml.

12. Procédé de la revendication 2, où le schéma thérapeutique comprend un inhibiteur de RAF ou un inhibiteur de MEK.

13. Procédé de la revendication 2, où le schéma thérapeutique comprend le vémurafénib ou le dabrafénib.
